# EUROPEAN PATENT SPECIFICATION

(11) **EP 1 285 057 B1**
(45) Date of publication and mention of the grant of the patent: **31.05.2006**
(21) Application number: 01930948.3
(22) Date of filing: 30.04.2001
(51) Int. Cl.: C12N 5/02, A61F 2/36

(54) **ISOLATION OF MESENCHYMAL STEM CELLS AND USE THEREOF**
ISOLIERUNG VON MESENCHYMALEN STAMMZELLEN UND DEREN VERWENDUNG
ISOLEMENT DE CELLULES SOUCHES MESENCHYMATEUSES ET UTILISATIONS CORRESPONDANTES

(30) Priority: 28.04.2000 US 200427 P
(43) Date of publication of application: 26.02.2003
(73) Proprietor: CHILDREN'S MEDICAL CENTER CORPORATION, Boston Massachusetts 02115 (US)
(72) Inventor: ATALA, Anthony, Weston, MA 02193 (US)
(74) Representative: Ouzman, Beverley Nicola Claire
(86) International application number: PCT/US2001/013891
(87) International publication number: WO 2001/083709

(56) References cited:
- WO-A-99/22747
- US-A- 5 942 225
- YOUNG ET AL.: 'Mesenchymal Stem Cells Reside Within the Connective Tissues of Many Organs' DEVELOPMENTAL DYNAMICS vol. 202, 1995, pages 137 - 144, XP002942312
- PITTENGER ET AL.: 'Multilineage Potential of Adult Human Mesenchymal Stem Cells' SCIENCE vol. 284, 02 April 1999, pages 143 - 147, XP002942313
- MACKAY ET AL.: 'Chondrogenic Differentiation of Cultured Human Mesenchymal Stem Cells from Marrow' TISSUE ENGINEERING vol. 4, no. 4, 1998, pages 415 - 428, XP002942310

## Description

### FIELD OF THE INVENTION

The present invention is generally directed to isolation of mesenchymal stem cells and a method of differentiating these cells into various lineages.

### BACKGROUND OF THE INVENTION

One of the major challenges facing tissue engineering is having a reliable and easily reproducible cell source for harvesting and expansion. Embryonic tissues¹⁻³, bone marrow, and skeletal muscle⁴⁻⁶ have been identified as sources for the derivation of mesenchymal stem cells with defined pluripotential properties. Mesenchymal stem cells can replicate as undifferentiated cells and have the remarkable ability to differentiate into specific kinds of mesenchymal or connective tissues including osseous (bone), cartilagenous, adipose (fat), muscular, elasltic, and fibrous connective tissues. Although these cells are normally present at very low frequencies in bone marrow, a process for isolating, purifying and mitotically expanding the population of these cells in tissue culture has been described in U.S. Patent Nos. 5,486,359; 5,197,985; and 5,226,914.

Adult mesenchymal stem cells go through a sequence of proliferation, commitment, differentiation and maturation. In a healthy individual, these cells take care of the replacement and regeneration of human connective tissues.

The ability to grow mesenchymal stem cells from a reliable cell source is crucial to production of complex tissues and organs for the restoration of damaged or diseased tissue.

Skin tissue is composed of mesoderm and ectoderm. The mesodermal layer contains many different cell types, and most have a stromal phenotype. Although fibroblasts are ubiquitous and posess the same phenotypic properties in mammalian tissues and organs, their biological properties may differ, depending on their natural environment. While it is possible that most fibroblasts are "mature", incapable of transformation, there is evidence that there are "immature" fibroblasts (pluripotent mesenchymal stem cells) that are capable of transformation.¹⁶

The molecular and cellular basis of directing an "immature" connective tissue phenotype to form bone, fat and cartilage is a remarkable biological phenomenon with enormous implications for disorders related to ostegenesis (e.g. the control of bone regeneration and fracture healing), adipogenesis (obesity and obesity-induced diabetes), and chondrogenesis (e.g., tissue engineering for numerous cartilage-regeneration therapies including treatment of urinary incontinence).
WO-A-99/22747 describes a bonding composition comprising isolated chrondrocytes in a gel, and teaches that differentiated chrondrocytes are obtained from cartilage tissue or bone marrow.
US 5,942,225 refers to human mesenchymal stem cells isolated from bone marrow, dermis or periosteum but only discloses a method of isolating such cells from bone marrow. There is no mention that mesenchymal stem cells can be isolated from human connective tissue.

For the foregoing reasons and deficiencies of the current state of the art, there exists a need for mesenchymal stem cells and especially mesenchymal stem cells derived from a reliable source and capable of being differentiated into different lineages of these cells, such as osteogenic, adipogenic and chondrogenic lineages.

### SUMMARY OF THE INVENTION

The present invention is directed to isolation of mesenchymal stem cells, their isolation to form a reliable source, and their differentiation into osteogenic, adipogenic and chondrogenic lineages. In accordance with one aspect of the invention, mesenchymal stem cells are isolated from a dermal layer of human skin, and more particularly from a postnatal human foreskin. In another aspect of the present invention, the isolated mesenchymal stem cells are differentiated into osteogenic, adipogenic and chondrogenic lineages.

As aforesaid, the first aspect of the present invention provides a method for isolating human mesenchymal stem cells. The method comprises providing a specimen of human connective tissue; holding the specimen in a sterile environment for at least 2 days (preferably for 2 to 10 days and most preferably for 3 to 6 days) at about 4°C; isolating a dermal layer from the specimen; contacting the dermal layer with collagenase, preferably collagenase I, to provide a collagenase cell suspension; collecting the supernatant from the collagenase cell suspension; centrifuging the supernatant to obtain a cell product, culturing the cell product under conditions to promote cell growth, and isolating mesenchymal stem cells from the culture. Preferably, the human connective tissue is obtained from a human skin, more preferably, from human postnatal foreskin. Preferably, the cells are subcultured using trypsin and EDTA for 5 minutes at 37°C.

In another aspect of the invention, a human mesenchymal stem cell produced by the method described supra is disclosed.

In a third aspect of the invention, a method for obtaining osteoblasts is provided. The method comprises: providing a specimen of human connective tissue; holding the specimen in a sterile environment for 2-10 days at about 4°C; isolating a dermal layer from the tissue; contacting the dermal layer with collagenase to provide a collagenase cell suspension; collecting the supernatant from the cell suspension; centrifuging the supernatant to obtain a cell product, and culturing the cell product under conditions to promote cell growth in contact with dexamethasone, betaglycerophosphate and ascorbic acid-2-phosphate in amounts sufficient to induce human mesenchymal stem cells to undergo osteogenic differentiation.

In a fourth aspect of the invention, a method for obtaining adipocytes is provided. The method comprises: providing a specimen of human connective tissue; holding the specimen in a sterile environment for 2-10 days at about 4C; isolating a dermal layer from the tissue; contacting the dermal layer with collagenase to provide a collagenase cell suspension; collecting the supernatant from the cell suspension; centrifuging the supernatant to obtain a cell product, and culturing the cell product under conditions to promote cell growth in contact with dexamethasone, 3-isobutyl-1-methylxanthine, insulin, and indomethacin in an amount sufficient to induce human mesenchymal stem cells to undergo adipogenic differentiation.

In a fifth aspect of the invention, a method for obtaining chondrocytes is provided. The method comprises: providing a specimen of human connective tissue; holding the specimen in a sterile environment for 2-10 days at about 4C; isolating a dermal layer from the tissue; contacting the dermal layer with collagenase to provide a collagenase cell suspension; collecting the supernatant from the cell suspension; centrifuging the supernatant to obtain a cell product, and culturing the cell product under conditions to promote cell growth in contact with L-glutamine, MEM nonessential amino acids, 2-mercaptoethanol, dexamethasone, ascorbic acid-2-phosphate, and transforming growth factor 3 in an amount sufficient to induce human mesenchymal stem cells to undergo chondrogenic differentiation.

Other aspects of the invention are disclosed *infra.*

### BRIEF DESCRIPTION OF THE FIGURES

For a more complete understanding of the invention, reference should be made to the figures, in which:

Figure 1 depicts the effect of adipogenic medium on mesenchymal stem cells in light microscopy and Oil-O-Red staining. Cells were seeded at 3x10³ cells/cm² in 2 well plates in control medium for 4 days. Subsequently, plates were cultured either in adipogenic medium or in control medium for 16 days. Before air-drying, the chamber slides for Oil-O-Red staining, pictures from cultures were taken in light microscopy.

Figure 2A depicts the effect of osteogenic medium on undifferentiated fibroblast cultures in alkaline phosphatase (AP) staining. Cells were grown in either control medium or in osteogenic medium for 4, 8, and 16 days.

Figure 2B shows the average of AP production of cells from 2 patients, treated either with osteogenic medium or with control medium after 4, 8, and 16 days of culture. Star marked values at Day 16 time point represent AP production of mesenchymal stem cells from bone marrow. Values were measured in quadruplicate values for each patient. P-Nitrophenol production is equivalent to alkaline phosphatase production.

Figure 3A shows the effect on undifferentiated fibroblast cultures of osteogenic medium in von Kossa staining. Undifferentiated cells were seeded at 3x10³ cells/cm² in 35 mm dishes. Cells were grown either in control medium or in osteogenic medium for 4, 8, and 16 days.

Figure 3B shows the average of two patients for calcium deposition of cells with Osteogenic Medium (solid line) and cells with Control Medium (broken line) after 4, 8, and 16 days of treatment. Values were measured in quadruplicate values for each patient. Results were normalized to cell counts.

Figure 4A shows a RT-PCR upregulation of cbfa1 in osteogenic cells.

Figure 4B is a RT-PCR performed for ppar -2 showing upregualtion of transcription in cells treated with adipogenic medium. Lipoprotein lipase expression is strongly induced in adipogenic cells.

### DETAILED DESCRIPTION OF THE INVENTION

In the present invention, the postnatal human dermal tissue was used as a source of pluripotential cells. Particularly, the present invention provides a method for isolating mesenchymal stem cells from postnatal human tissue, and a method of differentiate the mesenchymal stem cells into osteogenic, adipogenic, and chondrogenic lineages.

Osteogenic induction of mesenchymal stem cells has been reported for bone marrow, skeletal muscle, and embryonic stem cells. Osteogenic differentiation can be determined from a change in phenotype, the production of alkaline phosphatase, mineralization, accumulation of calcium, and the expression of genes specific for osteogenic induction.^{5,8,13} A transcription factor that is important for osteoblastic differentiation is cbfal.^{16,17} Cbfa1 is also highly expressed in chondrocytes when becoming hypertrophic for bone formation.¹⁸ In one aspect of the present invention, we disclose, *infra,* how mesenchymal stem cells can be derived from postnatal dermal tissue (preferably, postnatal human foreskin tissue), and can be made to differentiate into an osteogenic lineage.

Adipogenic differentiation has been reported for postnatal mesenchymal stem cells and for C3/T10 cells, derived from embryonic tissue.¹⁴ Adipogenic differentiation can be determined with oil-O-red staining, which stains intracellular fat-filled vacuoles, expression of lipoprotein lipase in adipogenic cells, and over-expression or induction of expression of pparγ-2, which is the most specific marker for adipogenic induction.^{5,14,15,19} In another aspect of the present invention, we disclose *infra* how mesenchymal cells, derived from human skin (preferably postnatal human foreskin) can be made to differentiate into an adipogenic lineage. Although Pittenger et al. used the same markers in an attempt to demonstrate the potential of bone marrow mesenchymal stem cells to differentiate into an adipogenic lineage, they were unsuccessful in that they were able to produce adipocytes only, they were not able to induce fibroblasts from skin to become osteogenic and adipogenic.⁵

In yet another aspect of the invention, we disclose *infra* a method of differentiating mesenchymal stem cells derived from human skin (preferably, postnatal human foreskin) into chondrocytes.

Although markers for differentiated cells are widely used and recognized, stem cell markers are more difficult to define, and marker expression may depend on the pluripotential ability of the cells. For example, mesenchymal stem cells derived from bone marrow express endoglin (CD-105) and Thy-1 (CD-90), but do not express CD-34 and CD-133, which are expressed in hematopoetic mesenchymal stem cells⁵. The mesenchymal cells of the present invention show positive expression for CD-90 and CD-105, which is consistent with the expression seen with bone marrow derived stem cells.

### EXAMPLES

### Cell Preparation and Culture Methods:

Human foreskin specimens are obtained during routine circumcisions from children aged 6 months to 18 years. The foreskin tissues are kept in sterile containers at 4°C for 3-6 days and are rinsed in betadine for 5 minutes prior to processing. The dermal layers are isolated, sectioned into small pieces (1 mm³) and digested in 15 ml of a sterile phosphate buffered solution (PBS) containing 1.25 mg/ml Collagenase I (Worthington Biochemicals) for 2 hours at 37°C. After 2 hours. Subsequently, the supernatant is collected and centrifuged at 1000 RPM for 5 minutes to obtain a cell product. The resulting cell product is then resuspended in culture medium, plated on 100mm bacterial dishes (VWR scientific products, Willard, OH) and grown to confluency for 2 to 3 weeks at 95% humidity and 37°C.

The culture medium preferably consists of DMEM high glucose (GIBCO/BRL, Grand Island, NY) with 20% embryonic stem cell-certified fetal bovine serum (ES- FBS, GIBCO/BRL, Grand Island, NY), 1% antibiotics (GIBCO/BRL, Grand Island, NY), L-glutamine, (Sigma-Aldrich, St.Louis, MO), 100mM MEM nonessential amino acids (Sigma-Aldrich, St.Louis, MO), 0.55mM 2-mercaptoethanol (Sigma-Aldrich, St.Louis, MO), and 2000U/ml leukemia inhibitory factor (LIF, R&D Systems). The cells can then be subcultured using 0.25% trypsin containing 1 mM EDTA for 5 minutes at 37°C.

For cell characterization, mesenchymal cells are seeded in 8 well chamber slides (nunc.Nalge) at a seeding density of 2500 cells per well. After three days, immunocytochemistry on cell surface markers for mesenchymal stem cells [CD105 (Pharmingen International), CD90 (Santa Cruz Biotechnology, Inc.)] and hematopoetic stem cells [CD34 (Pharmingen International), CD133] is performed.

Mesenchymal stem cells, as controls, can be purchased from Clonetics and cultured as recommended by the manufacturer's instructions.

### Osteogenic Differentiation:

For the induction of osteogenic differentiation, the mesenchymal cells are seeded at 3000 cells/cm² in 24 well plates (Falcon), 35 mm and 60 mm plates and cultured in DMEM low glucose medium (GIBCOBRL, Grand Island, NY) with 10% fetal bovine serum (FBS, GIBCO/BRL, Grand Island, NY), 1% antibiotics (GIBCOBRL, Grand Island, NY), and osteogenic supplements [100nM dexamethasone (Sigma-Aldrich, St.Louis, MO), 10mM beta-glycerophosphate (Sigma-Aldrich, St.Louis, MO), and 0.05mM ascorbic acid-2-phosphate (Wako Chemicals)]. Control medium consists of DMEM high glucose with 15% ES-FBS, 1% antibiotics, L-glutamine, 100mM MEM nonessential amino acids, and 0.55mM 2-mercaptoethanol. Medium changes are preferably performed every 3 days.

### Adipogenic Differentiation:

For the induction of adipogenic differentiation, the cells are seeded at 3000 cells/cm² in two-well chamber slides (Nunc/Nalge) and 60mm plates and cultured in DMEM low glucose medium with 10% FBS, 1% antibiotics, and adipogenic supplements [1µM dexamethasone, 1mM 3-isobutyl-1-methylxanthine (Sigma-Aldrich, St.Louis, MO), 10µg/ml insulin (Sigma-Aldrich, St.Louis, MO), and 60µM indomethacin (Sigma-Aldrich, St.Louis, MO)]. All cells are preferably kept in control medium for four days, before adipogenic induction is started.

Control medium consists of DMEM high glucose with 15% ES-FBS, 1% antibiotics, L-glutamine, 100mM MEM nonessential amino acids, and 0.55mM 2-mercaptoethanol. The medium was changed every 3 days.

### Chondrogenic Differentiation:

For chondrogenic differentiation undifferentiated cells (e.g., in the amount of 0.25x10⁶ cells) are spun down in 15 ml polystyrene conical tubes (Falcon) and grown as a pelleted micromass with 0.5ml of a defined chondrogenic differentiation medium. The medium preferably consists of DMEM high glucose with 15% ES-FBS, 1% antibiotics, L-glutamine, 100mM MEM nonessential amino acids, 0.55mM 2-mercaptoethanol, 100nM dexamethasone, 50µg/ml ascorbic acid-2-phosphate, and 2.5ng/ml transforming growth factor 3 (TGF- 3 , R&D Systems). After 21 days, with medium changes every 48 hours, pellets are harvested and assayed as described below.

### Alkaline Phosphatase Assay:

Alkaline phosphatase enzyme cell activity is measured in quadruplicate cultures. After rinsing twice with warm PBS, the cells are incubated with 2-amino-2-methyl-1-propanol buffer, pH 10.3 (Sigma-Aldrich, St.Louis, MO, #221) with 40mg p-nitrophenyl phosphate (Sigma-Aldrich, St.Louis, MO, #104/40) added, at 37°C for 3 to 35 min. AP activity was calculated after measuring the absorbance of p-nitrophenol product formed at 405nm on a micro plate reader (Molecular Devices, Spectra Max Plus). As a standard, p-nitrophenol standard solution (Sigma-Aldrich, St.Louis, MO, #104-1) diluted in 2-amino-2-methyl-1-propanol buffer in concentrations from 0 to 100nMol p-nitrophenol, is used. Enzyme activity is expressed as nMol p-Nitrophenol/min/10⁶ cells.

### Histochemical Analyses:

Alkaline phosphatase activity is determined histologically, according to the manufacturer's instructions (Sigma-Aldrich Kit #85). Briefly, cells are fixed in a citrate-acetone solution. An alkaline-dye mixture (fast blue RR solution with naphthol AS-MX phosphate alkaline solution) is added to the cells in the 35 mm culture dishes. The cell cultures should be protected from direct light. Prior to viewing the cell cultures are rinsed with deionized water and air-dried.

The presence of mineralization in cell culture is determined by von Kossa staining. The cell culture plates are fixed with 10% formaldehyde for 1 hour, incubated with 2% silver nitrate solution for 10 min in the dark, washed thoroughly with deionized water, and then exposed to UV-light for 15 min.

The presence of adipose elements in cell culture is determined with Oil-O-Red staining. The 2-well chamber slides are washed in deionized water and air-dried. The cells are incubated with oil red O staining solution for 15 minutes, rinsed with 50% ethanol 3 times, rinsed with distilled water, counterstained with Gill's hematoxilin for 30 sec to 1 min, and rinsed in deionized water 3 to 4 times. Chamber slides are mounted with water-based mounting media.

### Cell Proliferation Assays:

Cells are seeded at 3000 cells/cm² in 24 well plates. Cell numbers are determined after 4, 8, and 16 days in quadruplicate values. For the first time point (4 days), the medium is removed from the 24 well plates. The cells are rinsed once with PBS/EDTA, and incubated with 0.2 ml trypsin/EDTA for 10 minutes at 37°C. The cells are re-suspended with trypsin/EDTA solution several times to avoid cell clusters, before being transferred to 9.8 ml of Isoton fluid (Coulter Counter). Cells are counted as recommended by the manufacturer's instructions (Coulter Counter).

An MTT assay is performed after 4, 8, and 16 days. 100µl of MTT reagent (Sigma-Aldrich, St. Louis, MO) is added to 1ml of medium for 3 hours. The cells are lysed and color is extracted with isopropanolol containing 0.1 M HCl. Extinction is read in a biorad reader at 570nm against 655nm. Results are expressed as a cell count.

### RT-PCR:

RNA is isolated from cultured cells and cell pellets with RNAzol reagent (Tel-Test Inc., Friendswood, TX) according to the manufacturer's protocol. RNA (2µg) is processed for c-DNA synthesis with Superscript II reverse transcriptase with random hexamers (Life Technologies, Rockville, MD). c-DNA is used for each PCR reaction, in a final volume of 30µl with 200nM dNTP, 10pM of each primer, 0.3U Taq-DNA-polymerase, reaction buffer, and MgCl₂ (Life Technologies, Rockville, MD), in a PTC-100 cycler (MJ-Research Inc., Watertown, MA). The cycling conditions consist of 94°C for 2 minutes, annealing at 63°C for 40 seconds, and elongation at 72°C for 1 minute. Cycle numbers preferably vary between 22 and 37 cycles. All primers can obtained from Life Technologies. PCR products are quantified with NIH Image 1.62.

### Immunocytochemistry:

Cells, can be grown on 8-well chamber slides (Nunc/Nalge) for 3 days were fixed in 2% formaldehyde for 5 minutes, in 4% formaldehyde for 5 minutes, and in ice-cold methanol. Cell layers are washed 3 times with PBS.

Cells are stained for CD34 (Pharmingen International), CD133 (Miltenyi Biotec, Bergisch Gladbach, Germany), CD 105 (Pharmingen International), and CD90 (Santa Cruz Biotechnology, Inc.).

### Cultivation and Characterization of Human Mesenchymal Stem Cell Cultures:

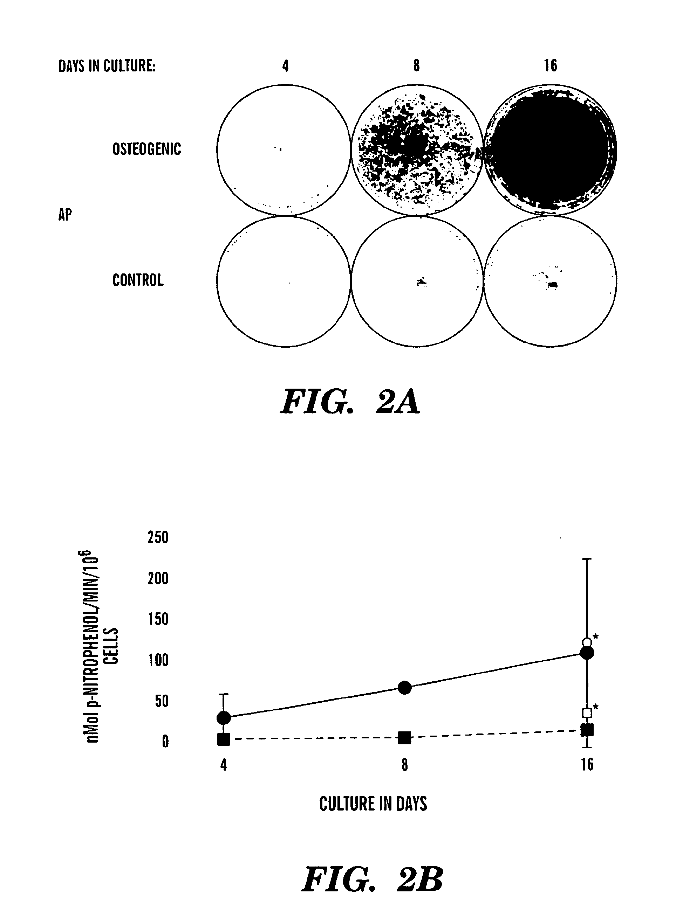

Cells are digested in collagenase type I, transferred into bacterial plates, and grown in a defined medium. After digestion only a few of the cells would attach to the plate surfaces. Under phase contrast microscopy, the cells may appear as short slightly spindle shaped cells. After 2 to 3 weeks in culture, cells can reach 70-80% confluence. Immunocytochemically, the cells express the mesenchymal stem cell markers CD-90 (Thy-1) and CD-105 (endoglin), and do not express either CD-34 or CD 1'33 (Figure 2).

### Induction of Human Mesenchymal Stem Cells into an Adipogenic Phenotype:

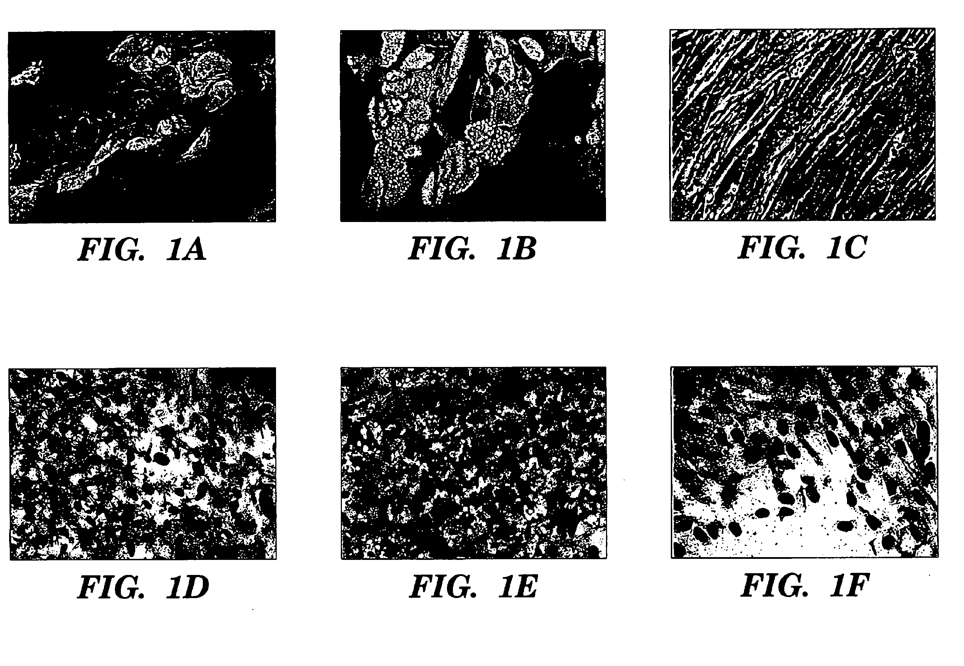

For adipogenic differentiation, the mesenchymal stem cells are treated with a defined medium with dexamethasone, insulin, indomethacin, and 3-isobutyl-1-methylxanthine. The cells tend to change their microscopic morphology from elongated to round. Within 8 days of exposure to the adipogenic medium, some cells may begin to form small intracellular translucent vacuoles that gradually fill the cytoplasm, along the cell membrane. After 16 days in culture, approximately 20-30% of the cells may be completely filled with these vacuoles. Oil-O-Red stains these areas red, consistent with the presence of adipose **(Figure 1)**. Control cells, grown in regular medium, tend not to show any signs of adipogenic differentiation and tend not to stain with Oil-O-Red at 8 or 16 days **(Figure 1)**.

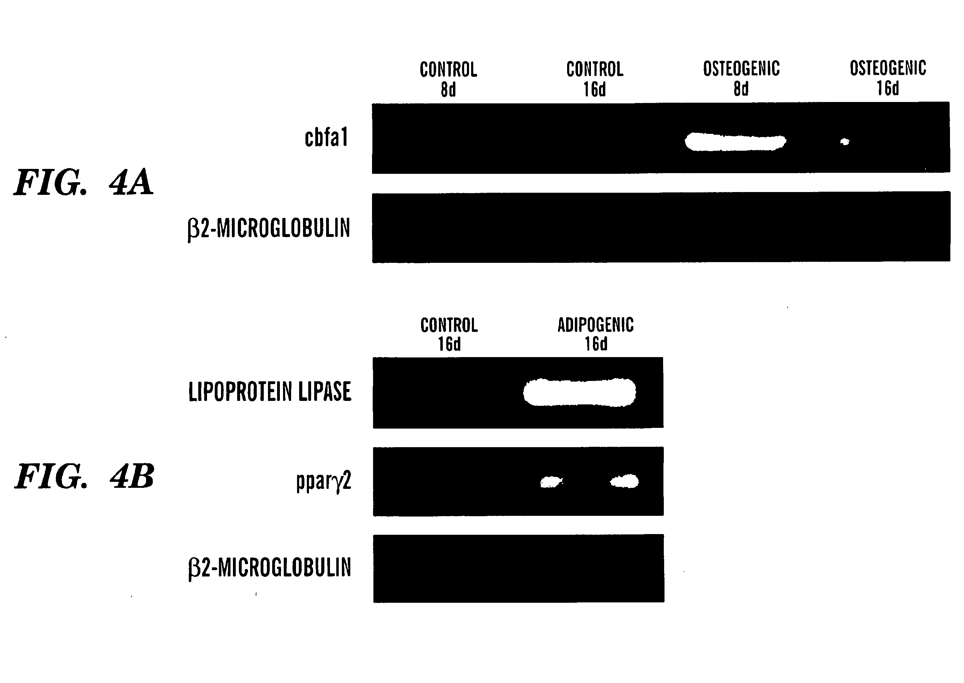

The adipogenic differentiation of the cells can be confirmed with RT-PCR for lipoprotein lipase (lpl), an adipocyte specific marker, and pparγ-2, a transcription factor highly expressed in adipocytes. To demonstrate equal amounts of RNA and c-DNA, β2-microglobulin as a housekeeping-gene can be used. After 16 days of treatment with adipogenic medium the cells tend to show strong expression of lpl and pparγ-2. In contrast, cells in the control medium do not express 1pl and only minimal pparγ-2 expression can be detected on day 16 **(Figures 4A-B).** These results suggest that undifferentiated cells from dermal tissue can be induced into an adipogenic lineage.

### Induction of Human Mesenchymal Stem Cells into an Osteogenic Phenotype:

Induction of human mesenchymal stem cells into an osteogenic phenotype is performed for 16 days. Phase contrast microscopy demonstrates that the mesenchymal cells, within 4 days of treatment, have a decreased spindle morphology and develop a round appearance with fingerlike excavations into the cell membrane.

Chemical staining for alkaline phosphatase shows a significant increase in the osteogenic medium treated cells by day 8 and 16 **(Figure 2A)**. The cells in the control medium do not show any significant difference in alkaline phosphatase staining at 8 or 16 days.

To confirm these findings, alkaline phosphatase activity in cells undergoing osteogenic induction is measured using a chemical assay **(Figure 2B)**. There is approximately a six-fold increase in alkaline phosphatase production in cells treated with osteogenic medium at 4 days compared to cells treated with control medium (30.6nM p-Nitrophenol/min/10⁶ cells and 8nM p-Nitrophenol/min/10⁶ cells, respectively). The osteogenic induced cells produce an average of 66.5nM p-Nitrophenol/min/10⁶ cells by day 8 and 109.1nM p-Nitrophenol/min/10⁶ cells by day 16. In contrast, the control cells produce an average of only 12.6nM p-Nitrophenol/min/10⁶ cells by day 16.

As an additional control, commercially available bone-marrow-derived mesenchymal stem cells, which have been reported to be strongly responsive for induction of alkaline phosphatase, are tested under the same conditions. Bone marrow cells can produce 120,2nM p-Nitrophenol/min/10⁶ cells at Day 16, similar to the mesenchymal stem cells derived from dermal tissue.

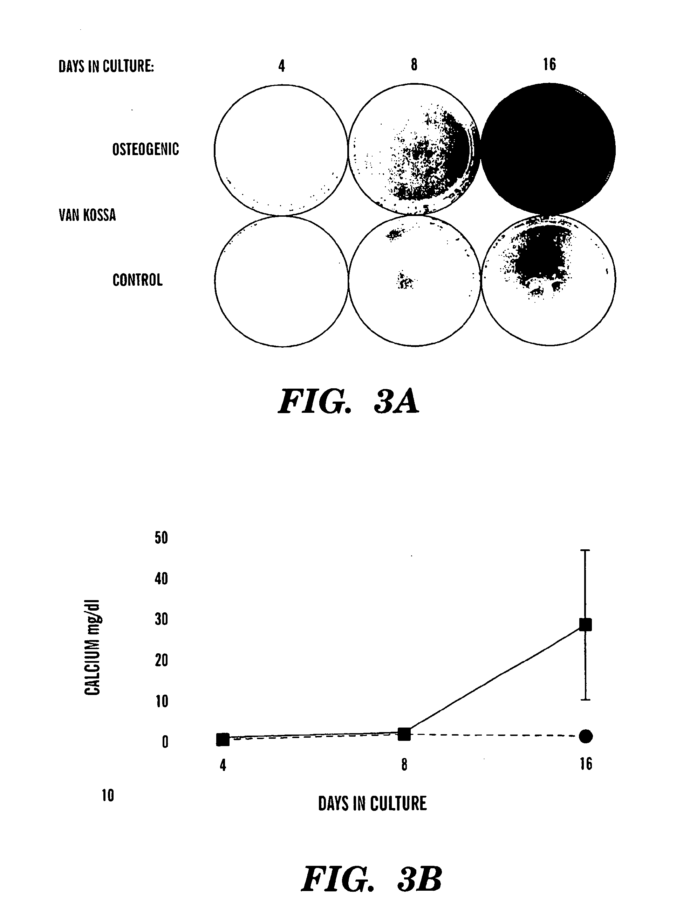

A major feature of osteogenic induction is the presence of cell-associated mineralization. In previous studies cell associated mineralization has been shown by using von Kossa staining and assays measuring the calcium content of cell cultures. Von Kossa staining of cells in the osteogenic media is strongly positive by day 16 **(Figure 3A)**. In contrast, cells in the control medium do not stain with silver nitrate after days 8 or 16.

To further test for mineralization, calcium deposition by the cells is measured **(Figure 3B).** Cells undergoing osteogenic induction show a significant increase in calcium precipitation after 16 days (28.5mg/dl). In contrast, cells grown in control medium do not show any increase in calcium accumulation (0.32 mg/dl) on day 16.

Undifferentiated mesenchymal cells are additionally cultured in 6-well dishes for 6 months. Osteogenic medium is added to half of the wells and control medium is added to the rest. The osteogenic medium cells form bone-like tissue in vitro, which stains positively with von Kossa, confirming calcification.

Expression of cbfal, a transcription factor highly expressed in osteoblasts and hypertrophic chondrocytes, is analyzed in cells using RT-PCR. To demonstrate equal amounts of RNA and c-DNA, β2-microglobulin, as a housekeeping-gene, can be used. Cbfal expression is highest in osteogenic differentiating cells after 8 days and decreased slightly at 16 days. There is nearly no expression of cbfal in the controls after 8 and 16 days **(Figures 4A-B)**.

The references cited herein, as set fort bove and below, are incorporated herein by reference in their entirety.

### References:

1. Pliard, A., Nifuji, A., Lamblin, D., Plee, E., Forest, C., and Kellermann, O. Controlled Conversion of an Immortilized Mesodermal Progenitor Cell Towards Osteogenic, Chondrogenic, or Adipogenic Pathways. J Cell Biol 130(6):1461-72 (1995)
2. Atkinson, B.L., Fantle, K.S., Benedict, J.J., Huffer, W.E., and Gutierrez-Hartmann, A. Combination of Osteoinductive Bone Proteins Differentiates Mesenchymal C3H/10T1/2 Cells Specifically to the Cartilage Lineage. J Cell Biochem 65:325-339 (1997)
3. Pinney, D.F., Emerson, Jr., C.P. 10T1/2 Cells: An In vitro Mode for Molecular Genetic Analysis of Mesodermal Determination and Differentiation. Environ Health Perspect 80:221-227 (1989)
4. Mackay, A.M., Beck, S.C., Murphy, J.M., Barry, F.P., Chichester, C.O., and Pitteneger, M.F. Chondrogenic Differentiation of Cultured Human Mesenchymal Stem Cells from Marrow. Tissue Engineering 4(4):415-428 (1998)
5. Pittenger, M.F., Mackay, A.M., Beck, S.C., Jaiswal, R.K., Douglas, R., Mosca, J.D., Moorman, M.A., Simonetti, D.W., Craig, S., and Marshak, D.R. Multilineage Potential of Adult Human Mesenchymal Stem Cells. Science 284:143-147, (1999)
6. Angele, P., Kujat, R., Nerlich, M., Yoo., J.,Goldberg, V., and Johnstone, B. Engineering of Osteochondral Tissue with Bone Marrow mesenchymal Progenitor Cells in a Derivatized Hyaluronan-Gelatin Composite Sponge. Tissue Engineering 5(6):545-553 (1999)
7. Young, H.E., Mancini, M.L., Wright, R.P., Smith, J.C., Black, Jr., A.C., Reagan, C.R., and Lucas, P.A. Mesenchymal Stem Cells Reside Within the Connective Tissue of Many Organs Dev Dyn 202:137-144, (1995)
8. Lee, J.Y., Qu-Petersen, Z, Cao, B., Kimura, S., Jankowski, R., Cummins, J., Usas, A., Gates, C., Robbins, P., Wemig, G, Huard, J. Clonal Isolation of Muscle-derived Cells Capable of Enhancing Muscle Regeneration and Bone Healing. J Cell Biol 2000 Sep 4;150(5):1085-100
9. Bosch, P., Musgrave, D.S., Lee, J.Y., Cumrnins, J., Shuler, T., Ghivizzani, T.C., Evans, T., Robbins, T.D., Huard, J. Osteoprogenitor Cells within Skeletal Muscle. J Orthop Res 2000 Nov;18(6):933-44
10. Alberts, B., Bray, D., Lewis, J., Raff, M., Roberts, K., Watson, J.D. Molecular Biology of the Cell (Second Edition), Chapter 17, pp. 986-988 (1989)
11. Lee, S, Lumelsky, N., Studer, L., Auerbach, J.M., McKay, R.D. Efficient Generation of Midbrain and Hindbrain Neurons from Mouse Embryonic Stem Cells. Nature Biotech 2000 Jun Vol 18:675-79
12. Reubinoff, B.E., Pera, M.F., Fong, C., Trounson, A., Bongso, A. Embryonic Stem Cell Lines from Human Blastocytes: Somatic Differeniation In Vitro. Nature Biotech 2000 Apr Vol 18:399-404
13. Jaiswal, N., Haynesworth, S.E., Caplan, A.I., Bruder, S.P. Osteogenic Differentiation of Purified Culture-Expanded Human Mesenchymal Stem Cells In Vitro. J Cell Biochem 64:295-312 (1997)
14. Lehmann, J.M., Lenhard, J.M., Oliver, B.B., Ringold, G.M., Kliewer, S.A. Peroxisome Proliferator-activated Receptors α and y Are Activated by Indomethacin and Other Non-steroidal Ant-inflammatory Drugs. J Biol Chem 1997 Feb;272(6):3406-10
15. Rosen, E.D., Sarraf, P., Troy, A.E., Bradwin, G., Moore, K., Milstone, D.S., Spiegelman, B.M., Mortensen, R.M. PPAR gamma Is Required for the Differentiation of Adipose Tissue In Vitro and In Vivo. Mol Cell 1999 Oct;4(4):611-7
16. Komori, T., Yagi, H., Nomura, S., Yamaguchi, A., Sasaki, K., Deguchi, K., Shimizu, Y., Bronson, R.T., Gao, Y.H., Inada, M., Sato, M., Okamoto, R., Kitamura, Y., Yoshiki, S., Kishimoto, T. Targeted Disruption of Cbfal Results in a Complete Lack of Bone Forrnation Owing to Maturational Arrest of Osteoblasts. Cell 1997 May 30;89(5):677-80
17. Karsenty, G. Role of Cbfal in Osteoblast Differentiation and Function. Semin Cell Dev Biol 2000 Oct; 11(5):343-6
18. Enomoto, H., Enomoto-Iwamoto, M., Iwamoto, M., Nomura, S., Himeno, M., Kitamura, Y., Kishimoto, T., Komori, T. Cbfal Is a Positive Regulatory Factor in Chondrocyte Maturation. J Biol Chem 2000 Mar; 275(12):8695-8702
19. Jaiswal, R.K., Jaiswal, N., Bruder, S.P., Mbalaviele, G., Marshak, D.R., Pittenger, M.F. Adult Human Mesenchymal Stem Cell Differentiation to the Osteogenic or Adipogenic Lineage Is Regulated by Mitogen-activated Protein Kinase. J Biol Chem 2000 Mar; 275(13):9645-9652

## Claims

1. A method for obtaining human mesenchymal stem cells, the method comprising:
(a) providing a specimen of human connective tissue;
(b) holding the specimen in a sterile environment for at least 2 days at about 4°C;
(c) isolating a dermal layer from the specimen;
(d) contacting the dermal layer with collagenase to provide a collagenase cell suspension;
(e) collecting supernatant from the collagenase cell suspension;
(f) centrifuging the supernatant to obtain a cell product;
(g) culturing the cell product under conditions to promote cell growth; and
(h) isolating mesenchymal stem cells from the culture.

2. The method of Claim 1, wherein the specimen is held in a sterile environment for 3-6 days.

3. The method of either one of Claims 1 and 2, wherein the collagenase is collagenase I.

4. The method of any one of Claims 1 to 3, wherein the human connective tissue is obtained from human skin.

5. The method of Claim 4, wherein the human skin is foreskin.

6. A human mesenchymal stem cell obtainable by the method of Claim 1.

7. Method for obtaining osteoblasts, the method comprising the method of any one of Claims 1 to 6 and then subsequently culturing the cell product under conditions to promote cell growth in contact with dexamethasone, beta-glycerophosphate and ascorbic acid-2-phosphate in amounts sufficient to induce the human mesenchymal stem cells to undergo osteogenic differentiation.

8. A method for obtaining adipocytes, the method comprising the method of any one of Claims 1 to 6 and then subsequently culturing the cell product under conditions to promote cell growth in contact with dexamethasone, 3-isobutyl-1-methylxanthine, insulin, and indomethacin in an amount sufficient to induce the human mesenchymal stem cells to undergo adipogenic differentiation.

9. A method for obtaining chondrocytes, the method comprising the method of any one of Claims 1 to 6 and then subsquently culturing the cell product under conditions to promote cell growth in contact with in contact with L-glutamine, MEM nonessential amino acids, 2-mercaptoethanol, dexamethasone, ascorbic acid-2-phosphate, and transforming growth factor 3 in an amount sufficient to induce the human mesenchymal stem cells to undergo chondrogenic differentiation.

## Patentansprüche

1. Ein Verfahren zum Erhalten von menschlichen mesenchymalen Stammzellen, wobei das Verfahren Folgendes beinhaltet:
(a) Bereitstellen einer Probe menschlichen Bindegewebes;
(b) Halten der Probe in einer sterilen Umgebung für mindestens 2 Tage bei ungefähr 4 °C;
(c) Isolieren einer Hautschicht von der Probe;
(d) Kontaktieren der Hautschicht mit Kollagenase, um eine Kollagenase-Zellsuspension bereitzustellen;
(e) Sammeln von Überstand von der Kollagenase-Zellsuspension;
(f) Zentrifugieren des Überstands, um ein Zellprodukt zu erhalten;
(g) Kultivieren des Zellprodukts unter Bedingungen, die das Zellwachstum fördern; und
(h) Isolieren von mesenchymalen Stammzellen von der Kultur.

2. Verfahren gemäß Anspruch 1, wobei die Probe für 3-6 Tage in einer sterilen Umgebung gehalten wird.

3. Verfahren gemäß Anspruch 1 oder 2, wobei die Kollagenase Kollagenase I ist.

4. Verfahren gemäß einem der Ansprüche 1 bis 3, wobei das menschliche Bindegewebe von menschlicher Haut erhalten wird.

5. Verfahren gemäß Anspruch 4, wobei die menschliche Haut Vorhaut ist.

6. Eine menschliche mesenchymale Stammzelle, die durch das Verfahren von Anspruch 1 erhältlich ist.

7. Ein Verfahren zum Erhalten von Osteoblasten, wobei das Verfahren das Verfahren gemäß einem der Ansprüche 1 bis 6 und dann das nachträgliche Kultivieren des Zellprodukts unter Bedingungen, die das Zellwachstum in Kontakt mit Dexamethason, Beta-Glycerophosphat und Askorbinsäure-2-phosphat fördern, in Mengen, die ausreichend sind, um die menschlichen mesenchymalen Stammzellen dazu zu veranlassen, osteogene Differenzierung einzugehen, beinhaltet.

8. Ein Verfahren zum Erhalten von Adipozyten, wobei das Verfahren das Verfahren gemäß einem der Ansprüche 1 bis 6 und dann das nachträgliche Kultivieren des Zellprodukts unter Bedingungen, die das Zellwachstum in Kontakt mit Dexamethason, 3-Isobutyl-1-methylxanthin, Insulin und Indomethacin fördern, in einer Menge, die ausreichend ist, um die menschlichen mesenchymalen Stammzellen dazu zu veranlassen, adipogene Differenzierung einzugehen, beinhaltet.

9. Ein Verfahren zum Erhalten von Chondrozyten, wobei das Verfahren das Verfahren gemäß einem der Ansprüche 1 bis 6 und dann das nachträgliche Kultivieren des Zellprodukts unter Bedingungen, die das Zellwachstum in Kontakt mit L-Glutamin, MEM nichtessentiellen Aminosäuren, 2-Mercaptoethanol, Dexamethason, Askorbinsäure-2-Phosphat und transformierendem Wachstumsfaktor-3 fördern, in einer Menge, die ausreichend ist, um die menschlichen mesenchymalen Stammzellen dazu zu veranlassen, chondrogene Differenzierung einzugehen, beinhaltet.

## Revendications

1. Une méthode destinée à obtenir des cellules souches mésenchymateuses humaines, la méthode comportant le fait :
(a) de fournir un spécimen de tissu conjonctif humain ;
(b) de garder le spécimen dans un environnement stérile pendant au moins 2 jours à environ 4 °C ;
(c) d'isoler une couche dermique du spécimen;
(d) de mettre en contact la couche dermique avec de la collagénase pour fournir une suspension cellulaire de collagénase ;
(e) de recueillir du surnageant à partir de la suspension cellulaire de collagénase ;
(f) de centrifuger le surnageant pour obtenir un produit cellulaire ;
(g) de cultiver le produit cellulaire dans des conditions pour favoriser la croissance cellulaire ; et
(h) d'isoler des cellules souches mésenchymateuses de la culture.

2. La méthode de la revendication 1, dans laquelle le spécimen est gardé dans un environnement stérile pendant de 3 à 6 jours.

3. La méthode de l'une ou l'autre des revendications 1 et 2, dans laquelle la collagénase est de la collagénase 1.

4. La méthode de n'importe laquelle des revendications 1 à 3, dans laquelle le tissu conjonctif humain est obtenu à partir de peau humaine.

5. La méthode de la revendication 4, dans laquelle la peau humaine est du prépuce.

6. Une cellule souche mésenchymateuse humaine qui peut être obtenue par la méthode de la revendication 1.

7. Méthode destinée à obtenir des ostéoblastes, la méthode comportant la méthode de n'importe laquelle des revendications 1 à 6 puis le fait de cultiver par la suite le produit cellulaire dans des conditions pour favoriser la croissance cellulaire en contact avec de la dexaméthasone, du bêta-glycérophosphate et de l'acide ascorbique-2-phosphate en quantités suffisantes pour induire les cellules souches mésenchymateuses humaines à subir une différenciation ostéogénique.

8. Une méthode destinée à obtenir des adipocytes, la méthode comportant la méthode de n'importe laquelle des revendications 1 à 6 puis le fait de cultiver par la suite le produit cellulaire dans des conditions pour favoriser la croissance cellulaire en contact avec de la dexaméthasone, du 3-isobutyl-1-méthylxanthine, de l'insuline et de l'indométhacine en quantité suffisante pour induire les cellules souches mésenchymateuses humaines à subir une différenciation adipogène.

9. Une méthode destinée à obtenir des chondrocytes, la méthode comportant la méthode de n'importe laquelle des revendications 1 à 6 puis le fait de cultiver par la suite le produit cellulaire dans des conditions pour favoriser la croissance cellulaire en contact avec de la L-glutamine, des acides aminés non essentiels MEM, du 2-mercaptoéthanol, de la dexaméthasone, de l'acide ascorbique-2-phosphate, et de transformer le facteur de croissance 3 en une quantité suffisante pour induire les cellules souches mésenchymateuses humaines à subir une différenciation chondrogénique.
